# FASCICULE DE BREVET EUROPEEN

(11) **EP 2 650 582 B1**
(45) Date de publication et mention de la délivrance du brevet: **27.05.2015**
(21) Numéro de dépôt: 13161313.5
(22) Date de dépôt: 27.03.2013
(51) Int. Cl.: F16M 11/04, B25J 15/04, B25J 19/00, H01R 13/629, A61B 19/00, A61B 17/00

(54) **Bras de suspension pour appareil électrique, équipement électrique pour bloc opératoire**
Schwingarm für Elektrogerät, elektrische Ausstattung für Operationstrakt
Suspension arm for electrical appliance, electrical equipment for operating suite

(30) Priorité: 10.04.2012 FR 1253261
(43) Date de publication de la demande: 16.10.2013
(73) Titulaire: Maquet SAS, 45160 Ardon (FR)
(72) Inventeur: Ravalitera, Pierre, 41210 Neung Sur Beuvron (FR); Monpou, Adrien, 45770 Saran (FR)
(74) Mandataire: Prugneau, Philippe

(56) Documents cités:
- EP-A2- 0 967 692
- US-A1- 2007 142 970

## Description

### Domaine technique

L'invention concerne un bras de suspension pour appareil électrique notamment pour l'équipement de bloc opératoire, le bras de suspension comportant une tête multifonction pourvue de connecteurs mécanique et électrique principaux destinés à coopérer respectivement avec des connecteurs électrique et mécanique secondaires portés par l'appareil électrique afin d'assurer des liaisons mécanique et électrique amovibles entre l'appareil électrique et la tête multifonction.

L'invention concerne également un équipement électrique pour bloc opératoire comportant au moins un bras de suspension supportant un appareil électrique.

### Technique antérieure

Ce type de bras de suspension est utilisé pour suspendre, par exemple au plafond du bloc opératoire, un appareil électrique tel que par exemple un dispositif d'éclairage, un écran de contrôle, une caméra ou tout autre appareil adapté. Le bras est par exemple de type articulé de sorte à pouvoir déplacer et orienter l'appareil électrique dans toute position adaptée au travail du personnel médical. En général, les blocs opératoires sont équipés de plusieurs bras portés par un même support. On comprend aisément qu'il est primordial que les liaisons mécanique et électrique entre l'appareil électrique et le bras de suspension soient fiables. Il est parfois nécessaire de changer un ou plusieurs de ces appareils électriques, par exemple pour des opérations de maintenance ou pour mettre en place un appareil électrique spécifique. Pour ce faire, l'opérateur doit démonter tout ou partie du bras de suspension afin de débrancher et désolidariser l'appareil électrique du bras de suspension. Ceci représente une opération longue et complexe. Certains appareils électriques nécessitent une connexion électrique particulière. Dans ce cas, le bras de suspension doit donc être modifié pour recevoir cette connexion électrique particulière. Cette opération est contraignante ce qui limite la modularité de chaque bras de suspension.

La publication US 2007/142 970 décrit un bras de robot pour intervention chirurgicale dont l'extrémité est pourvue d'une tête démontable. Le bras et la tête sont pourvus de rainures et nervures complémentaires, transversale à l'axe principal du bras et permettant leur engagement réciproque pour lier mécaniquement la tête au bras.

La publication EP 0 967 692 décrit un connecteur électrique comportant une fiche logée dans un boitier par rapport auquel elle est mobile entre une position de retrait dans laquelle elle est escamotée dans le boitier et une position avancée dans laquelle elle est proéminente et permet le raccordement électrique.

Toutefois, ces dispositifs de l'art antérieur ne permettent pas d'apporter une réponse optimale aux besoins des utilisateurs.

### Exposé de l'invention

Le but de l'invention est de remédier à ces inconvénients en proposant un bras de suspension pour appareil électrique et un équipement électrique pour bloc opératoire, garantissant des liaisons mécanique et électrique fiables, dans un encombrement limité, autorisant un montage-démontage et un branchement, débranchement rapides, fiables et sans démontage compliqué du bras de suspension, permettant l'utilisation d'une multitude de fiches électriques de sorte à rendre le bras de suspension compatible avec une grande variété d'appareils électriques.

A cet effet, l'invention a pour objet un bras de suspension pour appareil électrique notamment pour l'équipement de bloc opératoire, le bras de suspension comportant une tête multifonction pourvue de connecteurs mécanique et électrique principaux destinés à coopérer respectivement avec des connecteurs électrique et mécanique secondaires portés par l'appareil électrique afin d'assurer des liaisons mécanique et électrique amovibles entre l'appareil électrique et la tête multifonction, **caractérisé en ce que** le connecteur mécanique principal comporte un boîtier principal fixe agencé pour définir une glissière principale transversale à l'axe longitudinal du bras de suspension et définissant un logement principal pourvu d'une ouverture frontale principale, en ce que le connecteur électrique principal comporte au moins une fiche électrique principale couplée à des moyens d'actionnement agencés pour la déplacer longitudinalement entre une position de retrait dans laquelle elle est escamotée dans le logement principal et autorise l'engagement des connecteurs mécaniques principal et secondaire et une position avancée dans laquelle elle est au moins en partie proéminente par l'ouverture frontale principale de sorte à pouvoir s'engager avec le connecteur électrique secondaire et raccorder électriquement entre eux les connecteurs électriques principal et secondaire, en ce que la fiche électrique principale est prolongée par au moins un doigt de guidage s'étendant perpendiculairement à l'axe longitudinal, le doigt de guidage étant agencé pour traverser une rainure longitudinale prévue dans la paroi du boîtier principal et circuler dans une rainure de came ménagée dans une came prévue pivotante par rapport au boîtier principal de sorte que le pivotement de la rainure de came par rapport au boîtier principal provoque le déplacement de la fiche électrique principale entre ses positions de retrait et avancée.

L'idée à la base de l'invention est de prévoir une liaison mécanique par engagement transversal, couplée à une liaison électrique par engagement longitudinal. Cette bidirectionnalité des liaisons les rend plus fiables. Ainsi, la fixation et le branchement électrique sont obtenus par deux opérations distinctes. Les connecteurs assurant chaque liaison sont donc dissociés les uns des autres et optimisés pour assurer au mieux chacune des liaisons mécanique et électrique. De plus, la liaison électrique est réalisée dans un second temps par rapport à la liaison mécanique, donc après s'être assuré que la liaison mécanique est fiable. Il y a donc peu de risque, lors du branchement, de détériorer les connecteurs électriques par un déplacement inopiné entre l'appareil électrique et le bras de suspension. Enfin, la dissociation des liaisons électrique et mécanique permet, sans affecter l'efficacité des connecteurs mécaniques, de pouvoir changer les connecteurs électriques pour les adapter à des appareils électriques spécifiques.

Le bras de suspension selon l'invention peut avantageusement présenter les particularités suivantes :
- la glissière principale comporte une première paire de portions de glissière principale orientées à l'opposé l'une de l'autre et une seconde paire de portions de glissière principale orientées l'une vers l'autre, les première et seconde paires de portions de glissière principale étant décalées longitudinalement et transversalement l'une de l'autre et séparées l'une de l'autre part le logement principal ;
- la came est agencée pour pouvoir être manoeuvrée de l'extérieur du boîtier principal ;
- la fiche électrique principale est agencée pour pouvoir être désolidarisée du boîtier principal, et l'ouverture frontale est dimensionnée pour autoriser l'extraction complète de la fiche électrique principale du logement principal ;
- la tête multifonction est prévue pivotante par rapport à un axe principal prévu à l'extrémité du bras de suspension et sensiblement perpendiculaire à l'axe longitudinal ;
- le bras de suspension comporte un carter ouvert frontalement en regard du logement principal et agencé pour envelopper le boîtier principal.

L'invention s'étend à un équipement électrique pour bloc opératoire comportant au moins un bras de suspension supportant un appareil électrique, **caractérisé en ce qu'il** comporte un bras de suspension tel que décrit précédemment et au moins un appareil électrique pourvu d'un connecteur mécanique secondaire formant une glissière secondaire apte à coopérer avec la glissière principale pour assurer la fixation de l'appareil électrique à la tête multifonction et au moins un connecteur électrique secondaire apte à coopérer avec le connecteur électrique principal pour assurer le raccordement électrique de l'appareil électrique au bras.

L'appareil électrique peut comporter un boîtier secondaire définissant un logement secondaire logeant la fiche électrique secondaire et pourvu d'une ouverture frontale secondaire apte à autoriser le passage de la fiche électrique principale entre ses positions de retrait et avancée, la glissière secondaire comportant une première paire de portions de glissière secondaire orientées l'une vers l'autre apte à coopérer avec la première paire de portions de glissière principale, et une seconde paire de portions de glissière secondaire orientées à l'opposé l'une de l'autre apte à coopérer avec la seconde paire de portions de glissière principale, les première et seconde paires de portions de glissière secondaire étant décalées longitudinalement et transversalement l'une de l'autre et séparées l'une de l'autre part le logement secondaire.

### Présentation sommaire des dessins

La présente invention sera mieux comprise et d'autres avantages apparaîtront à la lecture de la description détaillée d'un mode de réalisation pris à titre d'exemple nullement limitatif et illustré par les dessins annexés, dans lesquels :
- la figure 1 est une vue en perspective de l'extrémité d'un bras de suspension selon l'invention pourvu d'une tête multifonction et illustrant les connecteurs électrique et mécanique principaux de la tête multifonction ;
- les figures 2A à 2C sont des vues en perspective d'une partie de la tête multifonction et d'un équipement illustrant les étapes de solidarisation mécanique d'un appareil électrique sur la tête multifonction du bras de suspension selon l'invention avec coopération entre le connecteur mécanique principal et le connecteur mécanique secondaire ;
- les figures 3A et 3B sont des vue en coupe et vue partielle en coupe selon deux plans distincts du bras de suspension et de l'appareil électrique des figures 2A à 2C après solidarisation mécanique et avant raccordement électrique entre les connecteurs électriques principal et secondaire ;
- les figures 4A et 4B sont des vues similaires aux figures 3A et 3B après solidarisation mécanique et raccordement électrique entre les connecteurs électriques principal et secondaire.

### Description des modes de réalisation

Le bras de suspension selon l'invention est par exemple destiné à être utilisé dans un bloc opératoire pour suspendre des appareils électriques utilisés par le personnel médical au cours des interventions chirurgicales. Ces appareils électriques sont par exemple des écrans, des appareils d'éclairage, des caméras ou tout autre appareil électrique adapté. Sur les figures, l'appareil électrique n'est qu'en partie schématisé. Ce dernier peut être raccordé directement au bras ou par l'intermédiaire d'une pièce intermédiaire tel qu'illustré. Par souci de simplification, on utilisera le terme appareil électrique également pour la pièce intermédiaire.

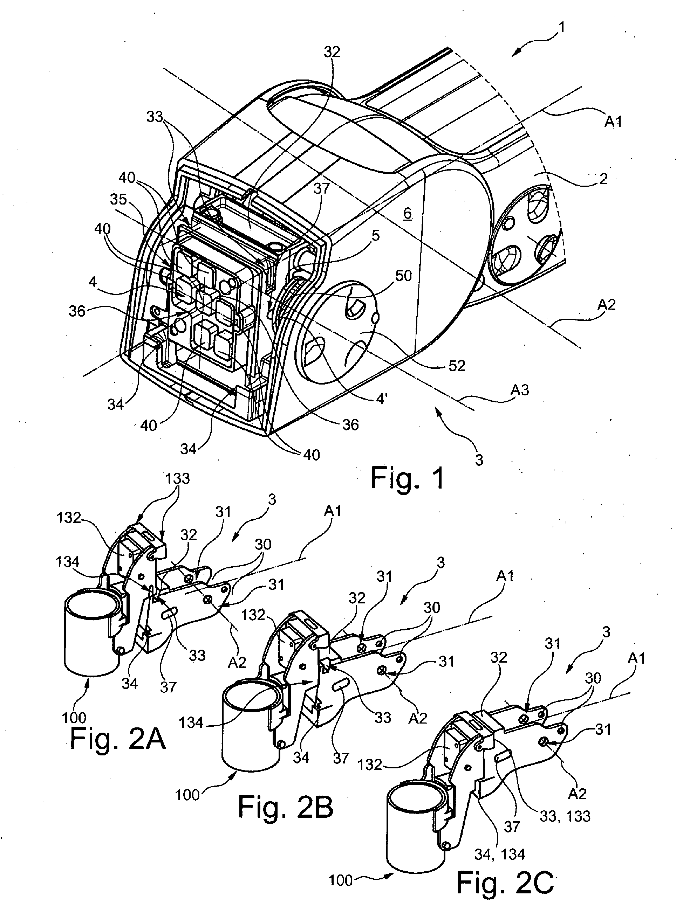

En référence à la figure 1, le bras de suspension 1 comporte une poutre 2 d'axe longitudinal A1 dont une première extrémité (non représentée) est par exemple fixée de manière articulée par rapport à un support (non représenté). La seconde extrémité de la poutre 2 est pourvue d'une tête multifonction 3. Cette seconde extrémité est traversée par un axe principal A2 sensiblement perpendiculaire à l'axe longitudinal A1 et définissant une liaison pivot principale autorisant le pivotement de la tête multifonction 3 par rapport à la poutre 2. Cette première liaison pivot peut être assurée par tout moyen connu. Ainsi, selon les moyens utilisés, l'inclinaison de la tête multifonction 3 peut être fixe par rapport à la poutre 2, ou variable de telle sorte que la tête multifonction 3 reste par exemple horizontale quelle que soit l'inclinaison de la poutre 2. Ce résultat peut par exemple être obtenu, par application du principe du parallélogramme déformable. En référence aux figures 2A à 4B, la tête multifonction 3 comporte deux étriers 30 traversés par des portées cylindriques 31 formant en partie la liaison pivot principale. Ces étriers 30 sont réunis par un boîtier principal 32 comportant des connecteurs principaux électrique 4 et mécanique 33, 34.

Dans sa partie supérieure, les bords externes du boîtier principal 32 définissent une première paire de portions de glissière principale 33 orientées à l'opposée l'une de l'autre. La première paire de portions de glissière principale 33 est apte à recevoir (comme illustré par les figures 2A à 2C détaillées plus loin) l'engagement d'une première paire de portions de glissière secondaire 133 orientées l'une vers l'autre et portées par l'appareil électrique 10. Dans sa partie inférieure, le boîtier principal 32 présente une forme en U dont les branches définissent une seconde paire de portions de glissières principale 34 orientées l'une vers l'autre. La seconde paire de portions de glissière principale 34 est apte à recevoir (comme illustré par les figures 2A à 2C détaillées plus loin) l'engagement d'une seconde paire de portions de glissière secondaire 134 orientées à l'opposé l'une de l'autre et portées par l'appareil électrique. Les première et seconde paires de portions de glissière principales 33, 34 sont disposées de part et d'autre du logement principal 35 décrit plu loin. La forme en U est décalée longitudinalement de sorte que la seconde paire de portions de glissière principale 34 est proéminente par rapport à la première paire de portions de glissière principale 33 en s'éloignant de la poutre 3. Les première et seconde paires de portions de glissière principale 33, 34 sont ouvertes transversalement vers le haut de telle sorte qu'elles autorisent l'engagement des première et seconde paires de portions de glissière secondaire 133, 134. Les première et seconde paires de portions de glissière principale 33, 34 définissent ainsi un connecteur mécanique principal apte à solidariser, sur la tête multifonction 3, un appareil électrique 100 pourvu d'un connecteur mécanique secondaire.

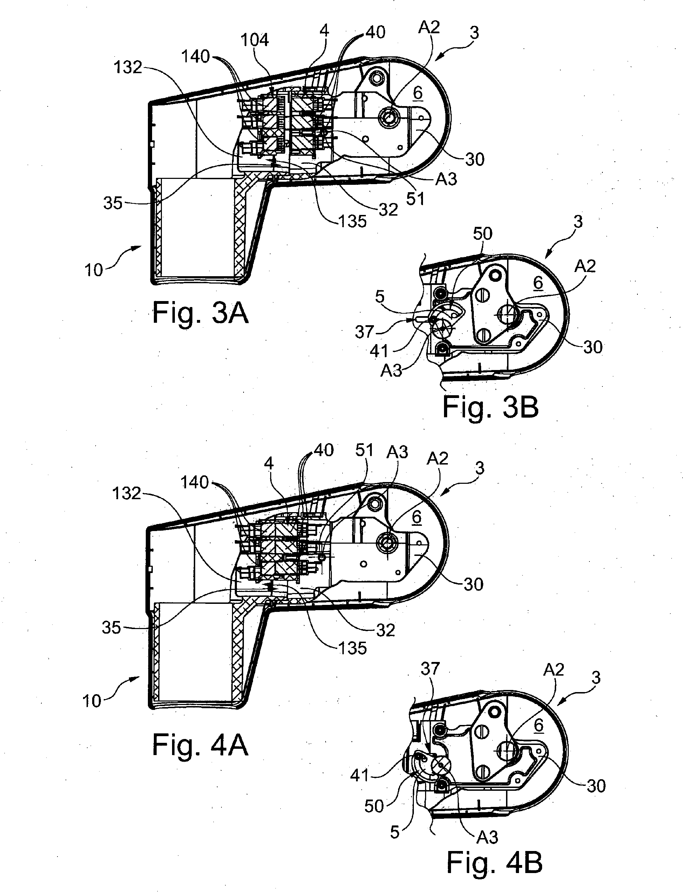

Le boîtier principal 32 définit par ailleurs un logement principal 35 apte à recevoir une fiche électrique principale 4 et pourvu d'une ouverture frontale principale prévue en regard de la fiche électrique principale 4. Dans l'exemple illustré, la fiche électrique principale 4 est de type « multiple » et comporte sept bornes électriques principales 40. La fiche électrique principale 4 peut bien entendu comporter un nombre de bornes électriques principales différent et/ou disposées différemment. Elle est montée coulissante dans le boîtier principal 32, guidée par une glissière longitudinale définie par l'agencement des bornes électriques principales 40 formant deux angles entrant, et des arêtes sortantes 36 ménagées dans le boîtier principal 32 et débouchant de l'ouverture frontale. Par l'intermédiaire de cette glissière longitudinale, la fiche électrique principale 4 est ainsi mobile entre une position de retrait (illustrée par les figures 1 et 3A) dans laquelle elle est escamotée, par exemple rasante ou en retrait par rapport à l'ouverture frontale principale, et une position avancée dans laquelle elle est proéminente par rapport à l'ouverture frontale principale. Ainsi, dans sa position de retrait, la fiche électrique principale 4 n'interfère pas avec la trajectoire des première et seconde paires de portions de glissière secondaire 133, 134 lors de leur engagement dans les premières et secondes portions de glissière principale 33, 34. De plus, dans sa position avancée, la fiche électrique 4 principale dépasse de l'ouverture frontale principale de sorte à pouvoir être engagée dans l'appareil électrique 100 pour raccorder électriquement la fiche électrique principale 4 à une fiche électrique secondaire 104 du connecteur électrique secondaire porté par l'appareil électrique 100. La fiche électrique principale 4 est bien entendu raccordée électriquement, par exemple à un câble (non représenté) logé dans la poutre 3. Le câble est par exemple un câble d'alimentation électrique et/ou un câble de transmission de signaux, par exemple de signaux « haute fréquence » tels que des signaux vidéo HD, des signaux informatiques. La fiche électrique principale 4 forme ainsi le connecteur électrique principal du bras de suspension 1.

Le boîtier principal 32 est entouré par un carter 6, ouvert en regard de l'ouverture frontale principale. Ce carter 6 protège notamment les doigts de guidage 41 décrits ci-après et les zones le guidant.

La fiche électrique principale 4 est pourvue de deux doigts de guidage 41 (dont l'un seulement est visible sur les figures 1, 3B et 4B) s'étendant latéralement, perpendiculairement à l'axe longitudinal A1. Ces doigts de guidage 41 traversent des rainures longitudinales 37, rectilignes, prévues dans les parois latérales du boîtier principal 32 et dans lesquelles ils peuvent librement circuler longitudinalement. De plus, les doigts de guidage 41 sont guidés dans des rainures de came 50 prévues dans des cames 5 montées pivotantes par rapport à un axe secondaire A3 porté par le boîtier principal 32. Les rainures de came 50 sont incurvées et présentent un rayon progressif par rapport à l'axe secondaire A3. Ainsi, lorsqu'une came 5 pivote autour de son axe secondaire A3, sa rainure de came 50 pivote et entraine le déplacement longitudinal du doigt de guidage 41 correspondant, guidé dans la rainure longitudinale 37, provoquant ainsi le déplacement de la fiche électrique principale 4 entre ses positions de retrait et avancée. Les deux cames 5 peuvent être reliées par une tige de commande 51 couplée à une molette d'actionnement 52 accessible à l'extérieur de la tête multifonction (visible sur la figure 1). Cette molette d'actionnement 52 peut être couplée à un levier (non représenté) facilitant son actionnement. Selon un mode de réalisation non représenté, le bras de suspension selon l'invention ne comporte qu'un seul doigt de guidage, une seule came et une seule rainure longitudinale. Selon un autre mode de réalisation non représenté, le bras de suspension comporte une seule came mais deux doigts circulant dans deux rainures longitudinales.

L'appareil électrique 100 comporte un boîtier secondaire 132 dont la partie supérieure présente une forme en U dont les branches définissent une première paire de portions de glissière secondaire 133 orientées l'une vers l'autre apte à recevoir l'engagement transversal de la première paire de portions de glissière principale 33. De plus, la partie inférieure définit une seconde paire de portions de glissière secondaire 134 orientées à l'opposé l'une de l'autre et apte à recevoir l'engagement transversal de la seconde paire de portions de glissière principale 34. Les premières et secondes paires de portions de glissière secondaire 133, 134 sont réparties de part et d'autre du logement secondaire 135 décrit plus loin. La forme en U est décalée longitudinalement de sorte que la première paire de portions de glissière secondaire 133 est proéminente par rapport à la seconde paire de portions de glissière secondaire 134 en s'éloignant de l'appareil électrique 100. Les première et seconde paires de portions de glissière secondaire 133, 134 sont ouvertes transversalement vers le bas de telle sorte qu'elles autorisent l'engagement des première et seconde paires de portions de glissière principale 33, 34. Elles définissent ainsi un connecteur mécanique secondaire apte à solidariser l'appareil électrique 100 sur la tête multifonction 3.

Le boîtier secondaire 132 définit par ailleurs un logement secondaire 135 logeant une fiche électrique secondaire 104 et pourvu d'une ouverture frontale secondaire prévue en regard de la fiche électrique secondaire 104. La fiche électrique secondaire 104 est fixe par rapport au logement secondaire 135. Dans l'exemple illustré, la fiche électrique secondaire 104 est de type « multiple » et comporte sept bornes électriques secondaires 140 aptes à coopérer avec les sept bornes électriques principales 40 de la tête multifonction 3. La fiche électrique secondaire 104 peut bien entendu comporter un nombre de bornes électriques secondaires différent. La fiche électrique secondaire 104 est prévue rasante ou en retrait de l'ouverture frontale secondaire pour ne pas interférer lors de l'engagement des première et seconde paires de portions de glissière principale 33, 34 et secondaire 133, 134. La fiche électrique secondaire 104 est bien entendu raccordée électriquement à la partie active de l'appareil électrique 100 (non représentée). La fiche électrique secondaire 104 forme ainsi le connecteur électrique secondaire du bras de suspension 1.

Pour solidariser mécaniquement l'appareil électrique 100 au bras de suspension 1, comme illustré sur la figure 2A, on place l'appareil électrique 100 de sorte que les première et seconde paires de portions de glissière principale 33, 34 et les première et seconde paires de portions de glissière secondaires 133, 134 soient transversalement respectivement dans l'alignement les unes des autres, les ouvertures frontales principale et secondaire étant orientées l'une vers l'autre tout en étant décalées en hauteur l'une de l'autre. Ainsi, les ouvertures frontales principale et secondaire sont sensiblement alignées dans un même plan par exemple perpendiculaire à l'axe longitudinal A1. Selon l'exemple illustré, l'appareil électrique 100 est ainsi disposé au-dessus de la tête multifonction 3.

Comme illustré par la figure 2B, ensuite, on déplace l'appareil électrique 100 par rapport à la tête multifonction 3 selon la direction transversale T, les première et seconde paires de portions de glissière principale 33, 34 et les première et seconde paires de portions de glissière secondaire 133, 134 étant toujours dans l'alignement les unes des autres. Ainsi, les connecteurs mécaniques principal et secondaire s'engagent mutuellement, les ouvertures frontales principale et secondaire se frôlant pour que les fiches électriques principale 4 et secondaire 104 passent l'une devant l'autre.

Comme illustré par les figures 2C, 3A et 3B, la liaison mécanique est assurée lorsque les première et seconde paires de portions de glissière principale 33, 34 et secondaire 133, 134 sont en butée au fond les unes des autres. A ce stade, les ouvertures frontales principales et secondaires sont en regard les unes des autres, les bornes électriques principales 40 étant en regard des bornes électriques secondaires 140 mais distantes entre elle. La fiche électrique principale 4 est encore dans sa position de retrait, le doigt de guidage 41 étant à l'extrémité de la rainure de came 50 présentant le rayon le plus faible.

Comme illustré par les figures 4A et 4B, pour assurer la liaison électrique, la molette d'actionnement 52 est manoeuvrée en rotation, dans cet exemple dans le sens antihoraire. Cette manoeuvre provoque le pivotement de la came 5 comportant la rainure de came 50 qui déplace de doigt de guidage 41 dans la rainure transversale 37 jusqu'à ce que le doigt de guidage 41 atteigne l'extrémité de la rainure de came 50 présentant le rayon le plus grand. La fiche électrique principale 4 est ainsi déplacée de sa position de retrait à sa position avancée dans laquelle elle s'insère dans le logement secondaire 135 pour mettre en contact les bornes électriques principales 40 et les bornes électriques secondaires 140.

L'appareil électrique 100 peut être démonté à tout moment. Pour ce faire, on désengage la fiche électrique principale 4 de la fiche électrique secondaire 104 par actionnement de la molette d'actionnement 52 dans le sens horaire de sorte à déplacer la fiche électrique principale 4 de sa position avancée à sa position de retrait. Ensuite, on dégage transversalement l'appareil électrique 100 en faisant coulisser les premières portions de glissière principale 33, 34 et secondaire 133, 134 entre elles. Une fois l'appareil électrique 100 démonté il peut être remplacé par tout autre appareil électrique équivalent.

L'invention permet d'atteindre les objectifs précédemment mentionnés. En effet, le bras de suspension 1 selon l'invention permet ainsi d'obtenir de manière simple et fiable une liaison mécanique efficace par simple accrochage vertical. Une fois la liaison mécanique assurée, la liaison électrique peut être établie de manière simple et rapide par rotation de la molette d'actionnement 52.

Il va de soi que la présente invention ne saurait être limitée à la description qui précède d'un de ses modes de réalisation, susceptibles de subir quelques modifications sans pour autant sortir du cadre de l'invention. Ainsi, par exemple, selon une variante de réalisation non représentée, la came peut être actionnée au moyen d'un actionneur mécanique, pneumatique ou de tout autre actionneur adapté. De plus, la translation de la fiche électrique principale est obtenue par des moyens mécaniques équivalents tels que par exemple un système arbre-came.

## Revendications

1. Bras de suspension (1) pour appareil électrique (100) notamment pour l'équipement de bloc opératoire, ledit bras de suspension (1) comportant une tête multifonction (3) pourvue de connecteurs mécanique et électrique principaux (33, 34, 4) destinés à coopérer respectivement avec des connecteurs électrique et mécanique secondaires (133, 134, 104) portés par ledit appareil électrique (100) afin d'assurer des liaisons mécanique et électrique amovibles entre ledit appareil électrique (100) et ladite tête multifonction (3), **caractérisé en ce que** ledit connecteur mécanique principal (33, 34) comporte un boîtier principal (32) fixe agencé pour définir une glissière principale transversale à l'axe longitudinal (A1) dudit bras de suspension (1) et définissant un logement principal (35) pourvu d'une ouverture frontale principale, **en ce que** ledit connecteur électrique principal comporte au moins une fiche électrique principale (4) couplée à des moyens d'actionnement (41, 37, 50, 5, 51) agencés pour la déplacer longitudinalement entre une position de retrait dans laquelle elle est escamotée dans ledit logement principal (35) et autorise l'engagement desdits connecteurs mécaniques principal (33, 34) et secondaire (133, 134) et une position avancée dans laquelle elle est au moins en partie proéminente par ladite ouverture frontale principale de sorte à pouvoir s'engager avec ledit connecteur électrique secondaire (104) et raccorder électriquement entre eux lesdits connecteurs électriques principal et secondaire (4, 104), **en ce que** ladite fiche électrique principale (4) est prolongée par au moins un doigt de guidage (41) s'étendant perpendiculairement audit axe longitudinal (A1), ledit doigt de guidage (41) étant agencé pour traverser une rainure longitudinale (37) prévue dans la paroi dudit boîtier principal (32) et circuler dans une rainure de came (50) ménagée dans une came (5) prévue pivotante par rapport audit boîtier principal (32) de sorte que le pivotement de ladite rainure de came (50) par rapport audit boîtier principal (32) provoque le déplacement de ladite fiche électrique principale (4) entre ses positions de retrait et avancée.

2. Bras de suspension (1) selon au moins la revendication 1, **caractérisé en ce que** ladite glissière principale comporte une première paire de portions de glissière principale (33) orientées à l'opposé l'une de l'autre et une seconde paire de portions de glissière principale (34) orientées l'une vers l'autre, lesdites première et seconde paires de portions de glissière principale (33, 34) étant décalées longitudinalement et transversalement l'une de l'autre et séparées l'une de l'autre part ledit logement principal (35).

3. Bras de suspension (1) selon au moins la revendication 1, **caractérisé en ce que** ladite came (5) est agencée pour pouvoir être manoeuvrée de l'extérieur dudit boîtier principal (32).

4. Bras de suspension (1) selon au moins la revendication 1, **caractérisé en ce que** ladite fiche électrique principale (4) est agencée pour pouvoir être désolidarisée dudit boîtier principal (32) et **en ce que** ladite ouverture frontale est dimensionnée pour autoriser l'extraction complète de ladite fiche électrique principale (4) dudit logement principal (35).

5. Bras de suspension (1) selon l'une au moins des revendications précédentes, **caractérisé en ce que** ladite tête multifonction (3) est prévue pivotante par rapport à un axe principal (A2) prévu à l'extrémité dudit bras de suspension (1) et sensiblement perpendiculaire audit axe longitudinal (A1).

6. Bras de suspension (1) selon au moins la revendication 1, **caractérisé en ce qu'il** comporte un carter (6) ouvert frontalement en regard dudit logement principal (35) et agencé pour envelopper ledit boîtier principal (32).

7. Equipement électrique pour bloc opératoire comportant au moins un bras de suspension (1) supportant un appareil électrique (100), **caractérisé en ce qu'il** comporte un bras de suspension (1) selon au moins l'une des revendications précédentes et au moins un appareil électrique (100) pourvu d'un connecteur mécanique secondaire (133, 134) formant une glissière secondaire apte à coopérer avec ladite glissière principale pour assurer la fixation dudit appareil électrique (100) à la tête multifonction (3) et au moins un connecteur électrique secondaire (104) apte à coopérer avec ledit connecteur électrique principal (4) pour assurer le raccordement électrique dudit appareil électrique audit bras.

8. Equipement électrique selon la revendication 7, **caractérisé en ce que** ledit appareil électrique (100) comporte un boîtier secondaire (132) définissant un logement secondaire (135) logeant ladite fiche électrique secondaire (104) et pourvu d'une ouverture frontale secondaire apte à autoriser le passage de ladite fiche électrique principale (4) entre ses positions de retrait et avancée, **en ce que** ladite glissière secondaire comporte une première paire de portions de glissière secondaire (133) orientées l'une vers l'autre apte à coopérer avec ladite première paire de portions de glissière principale (33), et une seconde paire de portions de glissière secondaire (134) orientées à l'opposé l'une de l'autre apte à coopérer avec ladite seconde paire de portions de glissière principale (34), lesdites première et seconde paires de portions de glissière secondaire (133, 134) étant décalées longitudinalement et transversalement l'une de l'autre et séparées l'une de l'autre part ledit logement secondaire (135).

## Patentansprüche

1. Schwingarm (1) für Elektrogerät (100), insbesondere für die Ausstattung eines Operationstrakts, wobei der Schwingarm (1) einen Multifunktionskopf (3) aufweist, der mit mechanischen und elektrischen Hauptverbindern (33, 34, 4) ausgerüstet ist, die dazu bestimmt sind, jeweils mit von dem Elektrogerät (100) getragenen elektrischen und mechanischen Sekundärverbindern (133, 134, 104) zusammenzuwirken, um lösbare mechanische und elektrische Verbindungen zwischen dem Elektrogerät (100) und dem Multifunktionskopf (3) sicherzustellen, **dadurch gekennzeichnet,**
**dass** der mechanische Hauptverbinder (33, 34) ein festes Hauptgehäuse (32) umfasst, das ausgebildet ist zum Definieren einer quer zu der Längsachse (A1) des Schwingarms (1) verlaufenden Hauptgleitbahn und das eine mit einer frontalen Hauptöffnung ausgestattete Hauptaufnahme (35) definiert,
**dass** der elektrische Hauptverbinder wenigstens einen elektrischen Hauptstecker (4) umfasst, der an Betätigungsmittel (41, 37, 50, 5, 51) gekoppelt ist, die ausgebildet sind, um diesen längs zwischen einer zurückgezogenen Position, in welcher er in der Hauptaufnahme (35) eskamotiert ist und das Einsetzen der mechanischen Haupt- (33, 34) und Sekundärverbinder (133, 134) erlaubt, und einer vorgerückten Position, in welcher er wenigstens teilweise durch die frontale Hauptöffnung vorsteht, um mit dem elektrischen Sekundärverbinder (104) in Eingriff gelangen und elektrisch zwischen den elektrischen Haupt- und sekundärverbindern (4, 104) verbunden werden zu können,
**dass** der elektrische Hauptstecker (4) durch wenigstens einen sich senkrecht zu der Längsachse (A1) erstreckenden Führungsfinger (41) verlängert ist, wobei der Führungsfinger (41) ausgebildet ist zum Durchsetzen einer in der Wandung des Hauptgehäuses (32) vorgesehenen Längsnut (37) und zum Verfahren in eine Kurvenbahn (50), die in einer in Bezug auf das Hauptgehäuse (32) schwenkbar vorgesehenen Kurvenscheibe (5) ausgebildet ist derart, dass die Verschwenkung der Kurvenbahn (50) in Bezug auf das Hauptgehäuse (32) die Verlagerung des elektrischen Hauptsteckers (4) zwischen seinen zurückgezogenen und vorgerückten Positionen bewirkt.

2. Schwingarm (1) nach zumindest Anspruch 1, **dadurch gekennzeichnet, dass** die Hauptgleitbahn ein erstes Paar von einander abgekehrt orientierten Hauptgleitbahnabschnitten (33) und ein zweites Paar von einander zugekehrt orientierten Hauptgleitbahnabschnitten (34) umfasst, wobei die ersten und zweiten Paare von Hauptgleitbahnabschnitten (33, 34) längs und quer zueinander versetzt und durch die Hauptaufnahme (35) voneinander getrennt sind.

3. Schwingarm (1) nach zumindest Anspruch 1, **dadurch gekennzeichnet, dass** die Kurvenscheibe (5) ausgebildet ist, um von außerhalb des Hauptgehäuses (32) betätigt werden zu können.

4. Schwingarm (1) nach zumindest Anspruch 1, **dadurch gekennzeichnet, dass** der elektrische Hauptstecker (4) ausgebildet ist, um von dem Hauptgehäuse (32) abgelöst werden zu können und dass die frontale Öffnung zum Erlauben eines vollständigen Herausnehmens des elektrischen Hauptsteckers (4) aus dem Hauptgehäuse (35) bemessen ist.

5. Schwingarm (1) nach wenigstens einem der vorhergehenden Ansprüche, **dadurch gekennzeichnet, dass** der Multifunktionskopf (3) schwenkbar in Bezug auf eine am Ende des Schwingarms (1) vorgesehene und im Wesentlichen zu der Längsachse (A1) senkrechte Hauptachse (A2) vorgesehen ist.

6. Schwingarm (1) nach wenigstens Anspruch 1, **dadurch gekennzeichnet, dass** er eine frontal gegenüber der Hauptaufnahme (35) offene Einhausung (6)' umfasst und ausgebildet ist zum Umgeben des Hauptgehäuses (32).

7. Elektrische Ausstattung für Operationstrakt, umfassend wenigstens einen Schwingarm (1)', der ein Elektrogerät (100)' stützt, **dadurch gekennzeichnet, dass** sie einen Schwingarm (1)' gemäß wenigstens einem der vorhergehenden Ansprüche und wenigstens ein Elektrogerät (100)', das mit einem mechanischen Sekundärverbinder (133, 134)' versehen ist, der eine Sekundärgleitbahn bildet, die in der Lage ist, mit der Hauptgleitbahn zum Sicherstellen der Fixierung des Elektrogeräts (100)' mit dem Multifunktionskopf (3)' zusammenzuwirken, und wenigstens einen elektrischen Sekundärverbinder (104)', der in der Lage ist, mit dem elektrischen Hauptverbinder (4)' zum Sicherstellen der elektrischen Verbindung des Elektrogeräts mit dem Arm zusammenzuwirken, umfasst.

8. Elektrische Ausstattung nach Anspruch 7', **dadurch gekennzeichnet, dass** das Elektrogerät (100)' ein Sekundärgehäuse (132)' aufweist, das eine Sekundäraufnahme (135)' definiert, die den elektrischen Sekundärstecker (104)' aufnimmt und mit einer frontalen Sekundäröffnung ausgestattet ist, die in der Lage ist, den Durchgang des elektrischen Hauptsteckers (4)' zwischen seinen zurückgezogenen und vorgerückten Positionen zu erlauben, dass die Sekundärgleitbahn ein erstes Paar von einander zugekehrt orientierten Sekundärgleitbahnabschnitten (133)', die in der Lage sind, mit dem ersten Paar von Hauptgleitbahnabschnitten (33)' zusammenzuwirken, und ein zweites Paar von einander abgekehrt orientierten Sekundärgleitbahnabschnitten (134), die in der Lage sind, mit dem zweiten Paar von Hauptgleitbahnabschnitten (34)' zusammenzuwirken, umfasst, wobei die ersten und zweiten Sekundärgleitbahnabschnitte (133, 134) längs und quer zueinander versetzt und voneinander durch die Sekundäraufnahme (135)' getrennt sind.

## Claims

1. Suspending arm (1) for an electrical appliance (100) in particular for the equipment in an operating room, said suspending arm (1) comprising a multifunction head (3) provided with main mechanical and electrical connectors (33, 34, 4) intended to cooperate respectively with secondary mechanical and electrical connectors (133, 134, 104) of said electrical appliance (100) in order to ensure removable mechanical and electrical connections between said electrical appliance (100) and said multifunction head (3), **characterized in that** said main mechanical connector (33, 34) comprises a main fixed case (32) arranged so as to define a main slide transversal to the longitudinal axis (A1) of said suspending arm (1) and defining a main housing (35) provided with a main frontal opening, **in that** said main electrical connector comprises at least one main electrical plug (4) coupled with actuation means (41, 37, 50, 5, 51) arranged so as to move said plug longitudinally between a retracted position in which it is retracted into said main housing (35) and allows the engagement of said main (33, 34) and secondary (133, 134) mechanical connectors and an advanced position in which it protrudes at least partly through said main frontal opening so as to be engaged with said secondary electrical connector (104) and to electrically connect said main and secondary electrical connectors (4, 104) to one another, **in that** said main electrical plug (4) is prolonged by at least one guiding finger (41) extending perpendicularly to said longitudinal axis (A1), said guiding finger (41) being arranged so as to extend through a longitudinal groove (37) in the wall of said main case (32) and to move in a cam groove (50) in a cam (5) able to swivel with respect to said main case (32) so that the swiveling of said cam groove (50) with respect to said main case (32) causes said main electrical plug (4) to move between its retracted and advanced positions.

2. Suspending arm (1) according to at least claim 1, **characterized in that** said main slide comprises a first pair of slide main portions (33) opposite one another and a second pair of slide main portions (34) oriented towards one another, said first and second pairs of slide main portions (33, 34) being shifted longitudinally and transversely from one another and separated from one another by said main housing (35).

3. Suspending arm (1) according to at least claim 1, **characterized in that** said cam (5) is arranged so that it can be operated from the outside of said main case (32).

4. Suspending arm (1) according to at least claim 1, **characterized in that** said main electrical plug (4) is arranged so that it can be disassociated from said main case (32) and **in that** said frontal opening is dimensioned so as to allow the complete extraction of said main electrical plug (4) from said main housing (35).

5. Suspending arm (1) according to at least one of the preceding claims, **characterized in that** said multifunction head (3) is able to swivel with respect to a main axis (A2) at the end of said suspending arm (1) and substantially perpendicular to said longitudinal axis (A1).

6. Suspending arm (1) according to at least claim 1, **characterized in that** it comprises a casing (6) provided with a frontal opening facing said main housing (35) and arranged to jacket said main case (32).

7. Electrical equipment for an operating room comprising at least one suspending arm (1) supporting an electrical appliance (100), **characterized in that** it comprises a suspending arm (1) according to at least one of the preceding claims and at least one electrical appliance (100) provided with a secondary mechanical connector (133, 134) forming a secondary slide able to cooperate with said main slide for ensuring the fixation of said electrical appliance (100) to the multifunction head (3) and at least one secondary electrical connector (104) able to cooperate with said main electrical connector (4) for ensuring the electrical connection of said electrical appliance to said arm.

8. Electrical component according to claim 7, **characterized in that** said electrical appliance (100) comprises a secondary case (132) defining a secondary housing (135) for said secondary electrical plug (104) and provided with a secondary frontal opening able to allow the passage of said main electrical plug (4) between its retracted and advanced positions, **in that** said secondary slide comprises a first pair of secondary slide portions (133) oriented towards one another and able to cooperate with said first pair of main slide portions (33), and a second pair of secondary slide portions (134) opposite one another and able to cooperate with said second pair of main slide portions (34), said first and second pairs of secondary slide portions (133, 134) being shifted longitudinally and transversely from one another and separated from one another by said secondary housing (135).
